(19) Europäisches Patentamt / European Patent Office / Office européen des brevets

(11) **EP 3 804 770 B1**

(12) **EUROPEAN PATENT SPECIFICATION**

(45) Date of publication and mention of the grant of the patent:
**06.12.2023 Bulletin 2023/49**

(21) Application number: **19834837.7**

(22) Date of filing: **09.07.2019**

(51) International Patent Classification (IPC):
*A61L 27/20* (2006.01)    *A61L 27/52* (2006.01)
*C08L 5/08* (2006.01)    *A61L 27/54* (2006.01)
*A61K 8/73* (2006.01)    *A61Q 19/08* (2006.01)
*C08B 37/08* (2006.01)

(52) Cooperative Patent Classification (CPC):
(C-Sets available)
**A61L 27/54; A61K 8/735; A61L 27/20; A61L 27/52;
A61Q 19/08; C08B 37/0072; C08L 5/08;**
A61K 2800/91; A61L 2300/402; A61L 2400/06;
A61L 2430/34        (Cont.)

(86) International application number:
**PCT/KR2019/008435**

(87) International publication number:
**WO 2020/013580 (16.01.2020 Gazette 2020/03)**

(54) **HYALURONIC ACID FILLER HAVING HIGH LIFT CAPACITY AND LOW INJECTION FORCE**

HYALURONSÄURE-FÜLLSTOFF MIT HOHER LIFTING-KAPAZITÄT UND GERINGER INJEKTIONSKRAFT

PRODUIT DE COMBLEMENT À BASE D'ACIDE HYALURONIQUE PRÉSENTANT UNE CAPACITÉ DE COMBLEMENT ÉLEVÉE ET NE NÉCESSITANT QU'UNE FAIBLE FORCE D'INJECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.07.2018 KR 20180080210**

(43) Date of publication of application:
**14.04.2021 Bulletin 2021/15**

(73) Proprietor: **LG CHEM, LTD.**
**Yeongdeungpo-gu,
Seoul 07336 (KR)**

(72) Inventors:
• **JANG, Cheol**
  **Daejeon 34122 (KR)**
• **KIM, Ji Sun**
  **Daejeon 34122 (KR)**
• **SO, Jineon**
  **Daejeon 34122 (KR)**
• **LEE, Chang Hyun**
  **Daejeon 34122 (KR)**
• **LEE, Chung**
  **Daejeon 34122 (KR)**
• **REE, Hwayoun**
  **Daejeon 34122 (KR)**
• **JUNG, Hyun Tae**
  **Daejeon 34122 (KR)**
• **KIM, Goo Youn**
  **Daejeon 34122 (KR)**

(74) Representative: **Goddar, Heinz J.**
**Boehmert & Boehmert
Anwaltspartnerschaft mbB
Pettenkoferstrasse 22
80336 München (DE)**

(56) References cited:
WO-A1-2005/067994    WO-A1-2016/051219
WO-A1-2016/074794    KR-A- 20150 029 578
KR-A- 20170 118 105    KR-B1- 101 660 211
KR-B1- 101 672 562    KR-B1- 101 769 739
US-A1- 2010 255 068

(52) Cooperative Patent Classification (CPC): (Cont.)

C-Sets
**A61L 27/20, C08L 5/08**

**Description**

[TECHNICAL FIELD]

Cross-reference with related application(s)

The present application claims the benefit of priority based on Korean Patent Application No. 10-2018-0080210 filed on July 10, 2018.

The present invention relates to a hyaluronic acid filler, and more specifically, relates to a hyaluronic acid filler, which has properties of monophasic hyaluronic acid (HA) fillers and biphasic hyaluronic acid fillers simultaneously, and therefore is injectable at a low injection force with excellent lift capability, and a method for preparing thereof.

[BACKGROUND ART]

Tissue of human skin maintains its structure by extracellular matrix including proteins such as collagen, elastin and the like and glycosaminoglycans, but when defects of soft-tissue occur due to external shock, diseases or aging and so on, tissue enhancement such as soft-tissue enhancement has been used for medical and cosmetic purposes. This enhancement has been done surgically through plastic surgery, or has restored and corrected its shape in a non-surgical manner by injecting biological tissue or synthetic polymer chemicals into the area to increase and expand the volume of soft-tissue. Then, a substance, which is a component similar to skin tissue and is inserted into a specific site to expand soft-tissue, and thereby it expands the volume of cheeks, lips, breast, hips, etc. cosmetically and is used for anti-wrinkle or contour correction through reduction of fine wrinkles and deep wrinkles of skin, is called a soft tissue augmentation material, and it is generally called a dermal filler. The first generation dermal filler primally developed in connection with this filler includes products such as Zyderm and Zyplast prepared by extracting animal proteins derived from animals, that is, cows or pigs, etc., and Cosmoderm or Cosmoplast using human collagen, and the like, but they have been rarely operated recently because of short duration of the effect and inconvenience of performing a skin sensitization test one month before the procedure.

The second generation filler is a hyaluronic acid (hereinafter, also referred to as 'HA' ) filler and has longer duration of the effect than the collagen filler and consists of polysaccharides similar to human components, N-acetyl-D-glucosamine and D-glucuronic acid, and therefore it has less side effects and is easy to procedure and removal, and it is possible to maintain the skin moisture, volume and elasticity by attracting water, and thus it has suitable advantages as a filler for skin.

However, the hyaluronic acid itself shows a short half-life of only a few hours in the human body, and therefore there is a limitation in application, and thus researches have been conducted to increase the half-life (internal persistence) through crosslinking. For example, U.S. Patent No. 4,582,865 discloses a hyaluronic acid derivative crosslinked using divinylsulfone (DVS) as a crosslinking agent, and its hydrogel form has been marketed under the trade name Hyalfrom®, and U.S. Patent No. 5,827,937 discloses a method for preparing a hyaluronic acid derivative crosslinked product using a multifunctional epoxy compound as a crosslinking agent, and among them, Restylane®, a hydrogen form of a hyaluronic acid crosslinked product prepared using 1,4-butanediol diglycidyl ether (BDDE) as a crosslinking agent has been approved by U.S. FDA and is available worldwide as a filler for tissue enhancement.

Such a crosslinked hyaluronic acid filler includes a filler made of a single-phase (monophasic HA filler) and a filler made of dualphase (biphasic HA filler). The monophasic hyaluronic acid filler is prepared using a homogeneous liquid-like hydrogel comprising a crosslinked hyaluronic acid, and thus it generally has low elasticity and high cohesivity. Accordingly, when the monophasic hyaluronic acid filler is injected into skin, it is unlikely to deviate from the injected site, but there are problems that the injected form is not maintained for a long time and the shape (form) retention period is only about 2 months after the procedure.

The biphasic hyaluronic acid filler is prepared by mixing crosslinked hyaluronic acid particles alone or with non-crosslinked hyaluronic acid close to liquid (non-crosslinked hyaluronic acid (linear HA), and therefore it generally has high elasticity and low cohesivity. Accordingly, when the biphasic HA filler is injected, it may maintain its shape for a long time, but there is a problem that there is a high possibility of deviating from the injected site. A representative example of the biphasic HA filler is the aforementioned Restylane® (Galderma product).

As such, the monophasic HA filler and biphasic HA filler have advantages and disadvantages, respectively, and conventionally, there is an example in which the above fillers are mixed to have all the properties of the monophasic hyaluronic acid filler and biphasic hyaluronic acid filler, but in this case, the advantages of the monophasic hyaluronic acid filler and biphasic hyaluronic acid filler are rather reduced together, and therefore it is not suitable as a filler. Thus, there is a need for a filler which can maintain its shape for a long time while having a low possibility of deviating from the injected site.

In addition, when the physical properties of fillers are strong, there is a problem that the soft tissue augmentation capability becomes better thereby, but because of strong physical properties of fillers during a procedure, the injection force is increased, and therefore when a doctor operates on a patient, injection is difficult and also the injection force is uneven, and thus there is a difficulty in injecting in an accurate amount.

WO 2016/074794 describes a long-lasting injectable dermal filler in gel-form, comprising crosslinked hyaluronic acid and carboxymethyl cellulose. The hyaluronic acid is crosslinked with BDDE with a degree of modification, expressed as the ratio of the sum of mono- and double-linked BDDE-crosslinkers to the sum of hyaluronic acid disaccharide units, of 0.5% to 25%. The filler displays a low extrusion force and a high modulus of elasticity and high dynamic viscosity providing high volumizing capacity.

[DISCLOSURE]

[TECHNICAL PROBLEM]

The present invention is suggested to solve the above problems, and an object of the present invention is to provide a filler which has high viscoelasticity and adhesion as advantages of both monophasic hyaluronic acid fillers and biphasic hyaluronic acid fillers, and therefore has good lift capability at the injected site and thus has low possibility to deviate from the injected site and can maintain its shape for a long time and has a low injection force and thus is stably injectable.

Another object of the present invention is to provide a method for preparing such a filler.

[TECHNICAL SOLUTION]

The present invention is defined by the claims. Any "embodiment" or "example" which is disclosed in the description but is not covered by the claims should be considered as presented for illustrative purpose only. The present application has been invented to solve the above problems of the prior art, and has found that when a hyaluronic acid meets conditions such as specific molecular weight and degree of crosslinking, it has rheological properties of high cohesivity of monophasic fillers and high viscoelasticity of biphasic fillers simultaneously, and accordingly, it shows high lift capability and therefore it is easy to make it in a desired form and it is maintained for a desired period when injected into skin, etc., and filler components rarely move to other sites in a body and also injection force is excellently reduced, which can minimize pain during injection in patients, thereby completing the present invention.

Thus, as one aspect, the present invention relates to a hyaluronic acid filler showing properties of both monophasic fillers and biphasic fillers, thereby having high lift capability and low injection force, a prefilled syringe filled with the filler, a biomaterial for soft tissue augmentation comprising the filler, and an anti-wrinkle method comprising injecting it into biological tissue.

The hyaluronic acid (hereinafter, also referred to as 'HA' ) comprised in the filler of the present invention is a biopolymer in which repeating units consisting of N-acetyl-D-glucosamine and D-glucuronic acid are linearly connected, and it is present a lot in vitreous humor of eyes, synovial fluid of joint, cockscomb, and the like, and it has been widely used for medical and medical appliance such as ophthalmic surgical aids, joint function improving agents, drug delivery materials, eye drops, anti-wrinkle agents, or cosmetics, as it has excellent biocompatibility. Specifically, the hyaluronic acid comprised in the filler of the present invention may mean its salt in addition to the hyaluronic acid. The salt of the hyaluronic acid includes for example, inorganic salts such as sodium hyaluronic acid, potassium hyaluronic acid, calcium hyaluronic acid, magnesium hyaluronic acid, zinc hyaluronic acid, cobalt hyaluronic acid, and the like, and organic salts such as tetrabutyl ammonium hyaluronic acid, and so on all, but not limited thereto.

According to the claimed invention, the hyaluronic acid or its salt is crosslinked by an appropriate crosslinking agent.

The crosslinked hyaluronic acid derivative is prepared by crosslinking the above hyaluronic acid itself or its salt using a crosslinking agent. For crosslinking, a method using a crosslinking agent under an alkaline aqueous solution is used. The alkaline aqueous solution includes NaOH, KOH, preferably, NaOH aqueous solution, but not limited thereto. Then, in case of NaOH aqueous solution, it may be used at a concentration of 0.1N to 0.5N. The crosslinked hyaluronic acid comprised in the filler of the present invention shows high rheological properties (viscoelasticity, cohesivity) and lift capability and shows a low injection force, even if a crosslinking agent at a low concentration and a small amount is used.

The crosslinking agent is a compound comprising two or more of epoxy functional groups and it may vary, and as a preferable example, it includes butanediol diglycidyl ether (1,4-butanediol diglycidyl ether: BDDE), ethylene glycol diglycidyl ether (EGDGE), hexanediol diglycidyl ether (1,6-hexanediol diglycidyl ether), propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, bisepoxypropoxy ethylene (1,2-(bis(2,3-epoxypropoxy)ethylene), pentaerythritol polyglycidyl ether and sorbitol polyglycidyl ether, and the like, and among them, biepoxidebased 1,4-butanediol glycidyl ether is particularly preferable in aspect to low toxicity.

Herein, the term "degree of modification (MOD)" means a degree of modification of hyaluronic acid calculated by a numerical value (n) showing the number of moles of the crosslinking agent (for example, BDDE) bound to the whole hyaluronic acid molecule relative to the number of moles of N-acetyl-D-glucosamine in the unit of the hyaluronic acid (N-acetyl-D-glucosamine (GlcNAc) + D-glucuronic acid), and it is represented by the following Equation 1.

[Equation 1]

$$\text{Degree of modification} = \text{total number of moles of crosslinking agent} / \text{total number of moles of N-acetyl-D-glucosamine}$$

In the present invention, the filler is characterized in that such a degree of modification shows a range of 0.01 to 0.07 (1% - 7%), preferably, a range of 0.03 to 0.05 (3% ~ 5%), through crosslinking by the above crosslinking agent.

In addition, herein, the term "crosslinking ratio (CrR)" means the ratio of the number of moles of the crosslinked crosslinking agent relative to the number of moles of the total crosslinking agent, and it is represented by the following Equation 2.

[Equation 2]

$$\text{Crosslinking ratio} = \text{number of moles of crosslinked crosslinking agent} / \text{number of moles of total crosslinking agent}$$

In the present invention, the filler is characterized by showing a range of 0.1 to 0.2, preferably 0.14 to 0.17 through crosslinking. The hyaluronic acid filler according to the present invention has a characteristic of synergistically showing properties of monophasic and biphasic fillers at the same time by having the above MOD and CrR ranges.

Herein, the molecular weight of the crosslinked hyaluronic acid may be 2,500,000 Da or more, preferably 2,500,000 to 3,500,000 Da.

The term "elasticity" used herein means a property as solid when applying force to an object, that is, a property of changing the form when applying force, but returning to the original form when removing force. This elasticity is represented by storage modulus (G': elastic modulus), and its unit is pascal (Pa). In addition, the term, viscosity used herein means a property as liquid, that is, the quantity that describes a fluid' s resistance to flow. This viscosity may be represented by loss modulus (G": viscous modulus) and its unit is pascal (Pa).

The term, cohesivity used herein is attraction (adhesion) acting between filler particles, and it may cause filler particles to agglomerate together. The higher this cohesivity is, the bigger the force that can support tissue into which the filler is injected is. Commonly, the cohesivity may be indirectly measured by a compression test, and it is measured as resistance when compressed at a certain rate after loading on a rheometer, and its unit is gf (gram force).

In addition, the term, lift capability used herein is a capability to extend or restore the injected site, which can be represented as the product of storage elastic modulus and resistance during compression, and high lift capability means excellent tissue restore capability and less migration of fillers after injection. Generally, when the lift capability is high, physical properties of fillers are strong, and thus it shows a property of high injection force.

The term, injection force used herein represents the force required during filler injection.

Generally, the hyaluronic acid filler in a monophasic form shows a gel form with cohesivity (cohesive gel) and therefore it has low elasticity, but high cohesivity, and thus it exhibits the high injection force. The examples include Belotero® of Merz and Stylage® of Vivavy. In addition, the hyaluronic acid filler in a biphasic form shows a particle form and therefore it has a characteristic of high elasticity and low cohesivity, and to exhibit such high elasticity, the particle diameter is made large. The example includes Resyrane® of Garderma. However, such a biphasic hyaluronic acid filler requires a large force when it passes through a needle by a large particle diameter, resulting in a high injection force.

However, the hyaluronic acid filler according to the present invention is characterized by having a low injection force with high lift capability, by having high elasticity and cohesivity and accordingly, having properties of monophasic fillers and properties of biphasic fillers simultaneously. The hyaluronic acid filler according to the present invention shows the lift capability of 12,000 to 44,000 Pa*gf, when measured by a rheometer, and shows the injection force of 15 to 25 N, when measured by filling it in a 1ml glass syringe of Schott Company and using a 29G 1/2" needle of Terumo Company.

In addition, a hyaluronic acid particle, preferably, a crosslinked hyaluronic acid particle, in the hyaluronic acid filler according to the present invention, may show various shapes, but preferably, it may be in a sphere shape. Furthermore, the average diameter of this particle may be 300 to 400 $\mu$m. When having such a specific particle diameter range, it may have high lift capability due to excellent rheological properties and also show a low injection force.

In a preferable aspect, the hyaluronic acid filler according to the present invention may comprise a hyaluronic acid of 1 to 3 % by weight based on the total filler weight. In addition, the hyaluronic acid filler according to the present invention may further comprise water, an anesthetic or a combination thereof, in addition to the hyaluronic acid.

The anesthetic comprises one or more kinds of anesthetics, preferably, local anesthetics, known in the art, and the concentration of one or more of anesthetics is an effective amount for alleviating symptoms to be experienced when injecting a composition. The example of the anesthetic may be selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine and salts thereof. In one embodiment, the anesthetic may be lidocaine, for example, a form of lidocaine hydrochloride.

For the hyaluronic acid filler according to the present invention, the concentration of the anesthetic comprised in the filler may be about 0.1 % by weight to about 1.0 % by weight based on the total weight of the filler, for example, about 0.2 % by weight to about 0.5 % by weight of the composition. Preferably, it may be 0.3 % by weight.

The concentration of the anesthetic in the filler described herein may be therapeutically effective, and this means a concentration which is unharmful to a patient and is suitable for providing advantages in an aspect of convenience of procedures and compliance of patients.

In addition, the filler according to the present invention may further comprise a buffer solution, and the buffer solution may use anything used for preparation of hyaluronic acid hydrogels without limitation. A preferable example of the buffer solution may be a buffer solution comprising one or more kinds selected from the group consisting of citric acid, sodium monohydrogen phosphate, sodium dihydrogen phosphate, acetic acid, diethyl barbituric acid, sodium acetate, TAPS (tris(hydroxymethyl)methylamino)propanesulfonic acid), Bicine (2-bis(2-hydroxyethyl)amino)acetic acid), Tris (tris(hydroxymethyl)amino methane), Tricine (N-(2-hydroxy-1,1-bis(hydroxymethyl)ethyl)glycine), HEPES (4-(2-hydroxyethyl)-1-piperazine ethanesulphonic acid), TES (2-[[1,3-dihydroxy-2-(hydroxymethyl)propan-2-yl]amino]methanesulfonic acid) and PIPES (piperazine-N,N' -bis(2-ethanesulfonic acid), but not limited thereto. The content of the above components comprised in the buffer solution may be appropriately adjusted, but preferably, they may be comprised at a concentration of 0.3 to 2.0 g/L based on the buffer solution.

Moreover, the filler according to the present invention may further comprise a isotonic agent, and this isotonic agent may be used without limitation, as long as it is used for preparation of hyaluronic acid hydrogels. As a preferable isotonic agent, sodium chloride may be used, but not limited thereto. The content of the isotonic agent may be appropriately adjusted if necessary, and for example, it may be comprised in an amount of 7.0 to 9.0 g/L based on the buffer solution, but not limited thereto.

In one example according to the present invention, a buffer solution comprising sodium chloride, sodium monohydrogen phosphate and sodium dihydrogen phosphate in injection water was used.

As an additional aspect, the composition according to the present invention may further comprise acceptable components that can be comprised in preparation of a filler, in addition to the above components.

Furthermore, it is characterized in that a residual crosslinking agent in the hyaluronic acid filler having high viscoelasticity and cohesivity of the present invention is rarely comprised, and the residual crosslinking agent is preferably 0.5 ppm or less that is a detection limit.

This hyaluronic acid filler having high viscoelasticity and cohesivity according to the present invention may be very usefully used on a cosmetic or therapeutic purpose, by the present distinctive elastic property and cohesivity. As a specific example, this hyaluronic acid filler may be used for filling of biological tissue, anti-wrinkle by filling wrinkle, remodeling of the face, or restoration or increases of volume of soft-tissue such as lips, nose, hips, cheeks or breast, and the like, as a biomaterial for soft tissue augmentation. The hyaluronic acid filler may be administered in an administration form appropriate for such uses, and preferably, it may be an injection.

As other aspect, the present invention relates to a preparation method of the above hyaluronic acid filler having high viscoelasticity and cohesivity, comprising the following steps:

(a) preparing crosslinked hyaluronic acid hydrogels by adding a hyaluronic acid or its salt, a crosslinking agent to an alkaline aqueous solution and stirring and then reacting;
(b) cutting the hyaluronic acid hydrogels prepared in the step (a);
(c) preparing a buffer solution;

(d) washing and swelling the hyaluronic acid hydrogels cutted in the step (b) using the buffer solution prepared in the step (c);

(e) grinding the washed and swollen hyaluronic acid hydrogels in the step (d); and

(f) filling the hydrogels prepared in the step (e) into a syringe and then sterilizing.

The step (a) is a step of preparing crosslinked hyaluronic acid hydrogels by crosslinking reacting a hyaluronic acid or its salt in an alkaline aqueous solution using a crosslinking agent, and as the matters related to the hyaluronic acid or its salt, crosslinking agent, and crosslinked hyaluronic acid hydrogel, the same applies to those mentioned in the hyaluronic acid filler. The alkaline aqueous solution may use anything known as an alkaline aqueous solution suitable for preparation of hyaluronic acid hydrogels, without limitation, and for example, it may be NaOH, KOH, NaHCO$_3$, LiOH or a combination thereof, and preferably, it may be NaOH. The concentration of this alkaline aqueous solution may be 0.1 to 0.5 N, but not limited thereto. In addition, the concentration of the hyaluronic acid or its salt is a weight ratio of the hyaluronic acid or its salt based on the total weight of the mixture of the hyaluronic acid or its salt and alkaline aqueous solution, and it may be 10 to 25 % by weight, and the concentration of the crosslinking agent is 1 to 10 mol% based on the unit of the added hyaluronic acid or its salt of 1 mole. When the concentration of the crosslinking agent is used at a high concentration over the above range, a filler with excessively high elasticity is obtained, and when the concentration is less than the above range, the elasticity is excessibly low and therefore it is not possible to exhibit appropriate viscoelasticity. Specifically, the step (a) may be performed by mixing and stirring a hyaluronic acid or its salt, a crosslinking agent and an alkaline aqueous solution to mix homogeneously. It may be performed at a temperature during crosslinking that is a room temperature or more, preferably in a temperature range of 25 to 40 °C, for 15 to 22 hours.

The cutting process may use various known cutting processes of hyaluronic acid hydrogels. In one example, the crosslinked gel prepared after the reaction is obtained in a form of cake, and it may be divided into a half moon shape using a cutter such as a straw cutter and the like, and for example, it may be divided into six. Then, the cutting process may be performed by passing through (preferably 2 times or more) the gel divided as above using a crude cutter having constant intervals of blades.

The step (c) is a step of preparing a buffer solution used for washing and swelling the crosslinked hyaluronic acid hydrogels cutted in the step (b), and the buffer solution may be prepared by known preparation methods of a buffer solution. In addition, the buffer solution may further comprise an anesthetic additionally. In one specific embodiment of the present invention, the buffer solution was prepared by dissolving sodium monohydrogen phosphate hydrates, sodium dihydrogen phosphate hydrates, sodium chloride and lidocaine hydrochloride in a buffer tank filled with injection water. The buffer solution may be used without limitation as long as it is used for preparation of hyaluronic acid hydrogels. The example of this preferable buffer solution may be a buffer solution comprising one or more kinds selected from the group consisting of citric acid, sodium monohydrogen phosphate, sodium dihydrogen phosphate, acetic acid, diethyl barbituric acid, sodium acetate, TAPS (tris(hydroxymethyl)methylamino)propanesulfonic acid), Bicine (2-bis(2-hydroxyethyl)amino)acetic acid), Tris (tris(hydroxymethyl)amino methane), Tricine (N-(2-hydroxy-1,1-bis(hydroxymethyl)ethyl)glycine), HEPES (4-(2-hydroxyethyl)-1-piperazine ethanesulphonic acid), TES (2-[[1,3-dihydroxy-2-(hydroxymethyl)propan-2-yl]amino]methanesulfonic acid) and PIPES (piperazine-N,N' -bis(2-ethanesulfonic acid), but not limited thereto.

The step (d) is a step of washing and swelling the crosslinked hyaluronic acid hydrogels cutted in the step (b) with the buffer solution prepared in the step (c), and this step (d) may be repeated once or two times or more. When completing washing and swelling, the washing solution may be removed.

The step (e) is a step of grinding the washed and swollen hydrogels, and this grinding may be performed by various grinding methods, but preferably, it may be extrusion grinding.

As an additional aspect, after the step (e), the prepared hydrogel may be under a process such as sterilizing and/or degas and the like, and it may be quantitatively filled, sealed and sterilized in a suitable container, for example, a syringe.

[ADVANTAGEOUS EFFECTS]

The filler according to the present invention not only has low possibility to deviate from the injected sites and can maintain its shape for a long time, because of showing high lift capability, but also can minimize problems which can be caused when injecting a filler, for example, difficulty during operation of doctors due to a high injection force or pain of patients, by exhibiting a low injection force different from conventional fillers having high lift capability, and therefore it can be useful as a filler for restore of soft tissues such as cheeks, lips, breast, hips, and the like, or volume expansion, anti-wrinkle through reduction of fine wrinkles and deep wrinkles of skin, or contour correction.

[DETAILED DESCRIPTION OF THE EMBODIMENTS]

Hereinafter, the present invention will be described in more detail through examples. However, these examples are intended to illustrate the present invention exemplarily, and the scope of the present invention is not limited by these examples.

## Example 1: Preparation of the hyaluronic acid filler according to the present invention

For preparation of the hyaluronic acid filler according to the present invention, the following process was conducted.

Sodium hyaluronic acid, sodium hydroxide, and BDDE (1,4-Butanediol Diglycidyl Ether), having a molecular weight of 2.5 MDa to 3.5 MDa were weighed. The concentration of sodium hyaluronic acid during the reaction was 15wt%, and the mol% of BDDE was 4 mol% based on the unit of the added sodium hyaluronic acid (namely, N-acetyl -D-glucosamine and D- glucuronic acid) of 1 mol. Separately, a sodium hydroxide aqueous solution at a concentration of 0.25N was prepared and filtered. The weighed sodium hyaluronic acid, 0.25N sodium hydroxide aqueous solution and BDDE (1,4-Butanediol Diglycidyl Ether) were added to a mixer container and were mixed homogenously, and this mixer container was put in a constant-temperature waterbath and the crosslinking reaction was completed at a temperature of 30°C overnight. Then, the crosslinked hyaluronic acid hydrogels in which the reaction was completed were preliminarily ground. On the other hand, a buffer solution was prepared by dissolving salts and an anesthetic in a buffer tank filled with injection water at concentrations of sodium monohydrogen phosphate hydrates (12 hydrates) 1.26 g/L, sodium dihydrogen phosphate hydrates (monohydrates) 0.46 g/L, sodium chloride 7 g/L and lidocaine hydrochloride 3 g/L.

A part of the buffer solution was considered as the primary buffer solution and it was transferred to a washing tank through a 0.22$\mu$m filter, and the preliminarily cutted hyaluronic acid gel prepared earlier was transferred to the washing tank filled with the primary buffer solution and then was stirred to primarily wash and swell the hyaluronic acid gel, and then when swelling was completed, the washing solution was removed. Then, the secondary buffer solution was transferred into a washing tank through a 0.22$\mu$m filter, and then it was stirred to secondarily wash and swell the hyaluronic acid gel. When the washing and swelling were completed, the washing solution was removed. Then, the tertiary buffer solution 40L was transferred into a washing tank through a 0.22$\mu$m filter, and then it was stirred to tertiarily wash and swell the hyaluronic acid gel. The washing solution was removed as soon as the washing and swelling was completed.

After completing the tertiary washing and swelling, whether the pH of the washing solution was in the neutral range was confirmed, and after grinding the hyaluronic acid gel in which washing and swelling was completed, it was transferred to an extruder tank and was weighed, and so as to reach a desired weight of the gel weight, the buffer solution was added to correct the primary content. When the primary content correction was completed, the hyaluronic acid gel was extruded and ground in the extruder tank. Then, the ground hyaluronic acid gel was transferred to a sterile tank and was homogenized, and then the content was measured and the buffer solution was added to conduct the secondary content correction. The hyaluronic acid gel in which the content correction was completed was heat-treated at a temperature of 121°C or more, for 1 minute or more, and the hyaluronic acid gel before filling this was decompressed while stirring to conduct desaturation. Then, the hyaluronic acid gel in a fixed amount of filling was vacuumed/filled to each syringe and at the same time, it was stoppered with a rubber stopper. The filled syringes were steam sterilized in a final sterilizer at a temperature of 121°C or more for 8 minutes or more.

## Example 2: Preparation of the hyaluronic acid filler according to the present invention

For preparation of the hyaluronic acid filler according to the present invention, the following process was conducted.

Sodium hyaluronic acid, sodium hydroxide, and BDDE (1,4-Butanediol Diglycidyl Ether), having a molecular weight of 2.5 MDa to 3.5 MDa were weighed. The concentration of sodium hyaluronic acid during the reaction was 16wt%, and the mol% of BDDE was 4 mol% based on the unit of the added sodium hyaluronic acid (namely, N-acetyl -D-glucosamine and D- glucuronic acid) of 1 mol. Separately, a sodium hydroxide aqueous solution at a concentration of 0.25N was prepared and filtered. The weighed sodium hyaluronic acid, 0.25N sodium hydroxide aqueous solution and BDDE (1,4-Butanediol Diglycidyl Ether) were added to a mixer container and were mixed homogenously, and this mixer container was put in a constant-temperature waterbath and the crosslinking reaction was completed at a temperature of 30°C overnight. Then, the crosslinked hyaluronic acid hydrogels in which the reaction was completed were preliminarily cutted. On the other hand, a buffer solution was prepared by dissolving salts and an anesthetic in a buffer tank filled with injection water at concentrations of sodium monohydrogen phosphate hydrates (12 hydrates) 1.26 g/L, sodium dihydrogen phosphate hydrates (monohydrates) 0.46 g/L, sodium chloride 7 g/L and lidocaine hydrochloride 3 g/L.

A part of the buffer solution was considered as the primary buffer solution and it was transferred to a washing tank through a 0.22,um filter, and the preliminarily cutted hyaluronic acid gel prepared earlier was transferred to the washing tank filled with the primary buffer solution and then was stirred to primarily wash and swell the hyaluronic acid gel, and then when swelling was completed, the washing solution was removed. Then, the secondary buffer solution was transferred into a washing tank through a 0.22$\mu$m filter, and then it was stirred to secondarily wash and swell the hyaluronic acid gel. When the washing and swelling were completed, the washing solution was removed. Then, the tertiary buffer solution was transferred into a washing tank through a 0.22$\mu$m filter, and then it was stirred to tertiarily wash and swell the hyaluronic acid gel. The washing solution was removed as soon as the washing and swelling was completed.

After completing the tertiary washing and swelling, whether the pH of the washing solution was in the neutral range was confirmed, and after cutting the hyaluronic acid gel in which washing and swelling was completed, it was transferred to an extruder tank and was weighed, and so as to reach a desired weight of the gel weight, the buffer solution was added to correct the primary content. When the primary content correction was completed, the hyaluronic acid gel was extruded and ground in the extruder tank. Then, the ground hyaluronic acid gel was transferred to a sterile tank and was homogenized, and then the content was measured and the buffer solution was added to conduct the secondary content correction. The hyaluronic acid gel in which the content correction was completed was heat-treated at a temperature of 121°C or more, for 1 minute or more, and the hyaluronic acid gel before filling, this was decompressed while stirring to conduct degassing. Then, the hyaluronic acid gel in a fixed amount of filling was filled to each syringe and at the same time, it was stoppered with a rubber stopper. The filled syringes were steam sterilized in a final sterilizer at a temperature of 121°C or more for 10 minutes or more.

**Experimental example 1: Investigation of viscoelasticity properties of the hyaluronic acid filler prepared by the present invention**

For investigation of rheological properties of prepared Examples 1 and 2, analysis was conducted using a rheometer. For comparison with the filler of the present invention, viscoelasticity properties of commercially available filler preparations were also analyzed and compared. The commercially available filler preparations as comparative examples and analysis conditions were as follows.

<Comparative examples>

- Comparative example 1: Belotero Intense Lidocaine
- Comparative example 2: Teosyal PureSense Ultradeep Lidocaine
- Comparative example 3: Teosyal PureSense Ultimate Lidocaine
- Comparative example 4: Stylage L Lidocaine
- Comparative example 5: Stylage XL Lidocaine
- Comparative example 6: Juvederm Volift with Lidocaine
- Comparative example 7: Juvederm Voluma with Lidocaine.

<Analysis conditions>

Analysis conditions of Oscillatory and Rotational Rheometer

In case of storage elastic modulus (G') and complex viscosity ($\eta$*) test

(1) Test equipment: Rheometer (Anton Paar Ltd., MCR301)
(2) Frequency: 1 Hz
(3) Temperature: 25 °C
(4) Strain: 4 %
(5) Measuring geometry: 25 mm plate/plate
(9) Measuring gap: 1.0 mm

In case of resistance when compressed (Compression force)

(1) Test equipment: Rheometer (Anton Paar Ltd., MCR301)
(2) Gap: Initial position: 2.5 mm, Final position: 0.9 mm
(3) Speed: 0.8 mm/min
(4) Temperature: 25 °C
(5) Measuring geometry: 25 mm plate/plate
(9) Normal Force Measuring gap position: 1.5 mm

In case of injection force measurement

(1) Test equipment: Tensile tester (Stand: Mecmesin., MultiTest 2.5-xt, Load cell: ILC 100N)
(2) Speed: 10 mm/min
(3) Diplacement: 0-17 mm
(4) Temperature: 25 ° C
(5) Syringe: 1mL long OVS syringe (Schott Ltd.)
(6) Needle: 29G, 1/2", Thin wall (Terumo Ltd.)

Under the analysis conditions, the result values of the storage elastic modulus (G'), complex viscosity ($\eta^*$), Compression force, injection force, calculated lift capability (= storage elastic modulus*Compression force), and calculated numerical value of lift capability per injection force (Lift capability/injection force) were shown in Table 1.

[Table 1]

| Example/ Comparative example | Present invention | | Belotero | Teosyal | | Stylage | | Juvederm | |
|---|---|---|---|---|---|---|---|---|---|
| | Example 1 | Example 2 | Comparative example 1 Belotero Intense Lidocaine | Comparative example 2 Teosyal Ultradeep Lidocaine | Comparative example 3 Teosyal Ultimate Lidocaine | Comparative example 4 Stylage L Lidocaine | Comparative example 5 Stylage XL Lidocaine | Comparative example 6 Juvederm Volift with Lidocaine | Comparative example 7 Juvederm Voluma with Lidocaine |
| Concentration (mg/mL) | 20 | 20 | 25.5 | 25 | 22 | 24 | 26 | 17.5 | 20 |
| Storage Modulus (1 Hz) | 448 | 707 | 149 | 374 | 409 | 225 | 264 | 314 | 310 |
| Complex viscosity ($\times 10^4$ cP, 1 Hz) | 7.18 | 11.3 | 2.54 | 5.98 | 6.54 | 3.65 | 4.28 | 5.08 | 4.97 |
| Compression force (gf) | 36 | 44 | 71 | 53 | 52 | 55 | 65 | 20 | 24 |
| Maximum injection force (N, 10mm/min, 0-17mm, 29G) | 22 | 21 | 50 | 50 | 47 | 42 | 34 | 11 | 35 |
| Average injection force (N, 10mm/min, 8-15mm, 29G) | 20 | 19 | 48 | 31 | 34 | 38 | 32 | 10 | 25 |
| Lift capability | 16145 | 31391 | 10551 | 19862 | 21237 | 12354 | 17183 | 6373 | 7493 |
| Lift capability / maximum injection force | 741 | 1516 | 210 | 398 | 448 | 298 | 511 | 579 | 215 |
| Lift capability / average injection force | 795 | 1635 | 221 | 645 | 630 | 328 | 542 | 625 | 305 |

EP 3 804 770 B1

As can be seen in the Table 1, it can be confirmed that the hyaluronic acid hydrogel filler according to the present invention shows higher lift capability compared to other commercially available fillers. In case of Comparative examples 2 and 3 among Comparative examples, it may be shown that they show a little high lift capability, but it is determined that problems during operation such that it is may not constantly injected during operation or requires strong power to operate due to relatively high injection force and the like are relatively higher compared to the present invention. Accordingly, it can be confirmed that Examples 1 and 2 according to the present invention, which has the most and superiorly high ratio of lift capability to injection force that is a parameter showing physical properties which can show the best operation effects.

**Experimental example 2: Analysis of the particle size of the hyaluronic acid hydrogels according to the present invention**

In order to confirm the particle size of the hyaluronic acid hydrogels of Examples 1 and 2 and Comparative examples 1 to 7 and distribution, the following test was conducted. The corresponding result of this test was shown in Table 2.

<Analysis conditions>

Analysis conditions of Laser diffraction particle size analyzer
(1) Test equipment: Laser diffraction particle size analyzer (Malvern Ltd., Mastersizer 3000)
(2) Dispersant: 0.9% NaCl solution
(3) Stirrer rpm: 1,000
(4) Laser obscuration: 5-25 %

[Table 2]

| Example/ Comparative example | Present invention | | Belotero | Teosyal | | Stylage | | Juvederm | |
|---|---|---|---|---|---|---|---|---|---|
| | Example 1 | Example 2 | Comparative example 1 Belotero Intense Lidocaine | Comparative example 2 Teosyal Ultradeep Lidocaine | Comparative example 3 Teosyal Ultimate Lidocaine | Comparative example 4 Stylage L Lidocaine | Comparative example 5 Stylage XL Lidocaine | Comparative example 6 Juvederm Volift with Lidocaine | Comparative example 7 Juvederm Voluma with Lidocaine |
| Particle diameter, Dv (50) ($\mu$m) | 362 | 343 | 571 | 726 | 943 | 375 | 358 | 407 | 408 |

As can be seen in the Table 2, it can be confirmed that the hyaluronic acid filler according to the present invention show the particle diameter (Dv50) of 300 to 400 μm, while Comparative examples 1 to 3 and Comparative examples 6 and 7 has the relatively thicker particle diameter.

**Experimental example 3: Analysis of Degree of Modification of the hyaluronic acid hydrogels according to the present invention**

In order to confirm the degree of modification of the hyaluronic acid hydrogels of Examples 1 and 2 and Comparative examples 1 to 6, a test was performed under the following conditions. The result of this test was shown in Table 3.

<Analysis conditions>

Analysis conditions of Nuclear Magnetic Resonance
(1) Test equipment: FT-NMR System (Jeol Ltd., ECA500/ECZ400S),
(2) Pulse: 30 °
(3) Scans: 512
(4) Relaxation time (delay): 5 s
(5) Temperature: 25 ° C

[Table 3]

| Example/Comparative example | Present invention | | Belotero | Teosyal | | Stylage | | Juvedem | |
|---|---|---|---|---|---|---|---|---|---|
| | Example 1 | Example 2 | Comparative example 1 Belotero Intense Lidocaine | Comparative example 2 Teosyal Ultradeep Lidocaine | Comparative example 3 Teosyal Ultimate Lidocaine | Comparative example 4 Stylage L Lidocaine | Comparative example 5 Stylage XL Lidocaine | Comparative example 6 Juvederm Volift with Lidocaine | Comparative example 7 Juvederm Voluma with Lidocaine |
| Modification degree (%) | 3.3 | 3.5 | 8.5 | 14 | 16.5 | 7.8 | 7.8 | 6.3 | 6 |

As can be seen in the Table 3, it can be seen that the hyaluronic acid fillers of Examples 1 and 2 according to the present invention exhibit a low degree of modification despite of showing excellent physical properties as confirmed earlier, and this means that it is very biocompatible as a filler showing excellent physical properties can be provided even when using a small amount of crosslinking agent during preparation of a filler.

**Claims**

1. A hyaluronic acid hydrogel filler, comprising a hyaluronic acid or its salt, and showing lift capability of 12,000 to 44,000 Pa*gf as measured by a rheometer and an injection force of 15 to 25 N when measured by a tensile tester, in which the hyaluronic acid or its salt is crosslinked, wherein

   the filler has both properties of monophasic and biphasic hyaluronic acid fillers,
   a degree of modification (MOD) of the hyaluronic acid as represented by the following Equation 1 is in the range of 0.01 to 0.07 (1 to 7%):

$$[\text{Equation 1}]$$

$$\text{Degree of modification} = \text{total number of moles of crosslinking agent} \, / \, \text{total number of moles of N-acetyl-D-glucosamine},$$

   and
   a crosslinking ratio (CrR) as represented by the following Equation 2 is in the range of 0.1 to 0.2:

$$[\text{Equation 2}]$$

$$\text{Crosslinking ratio} = \text{number of moles of crosslinked crosslinking agent} \, / \, \text{number of moles of total crosslinking agent}.$$

2. The hyaluronic acid hydrogel filler according to claim 1, wherein the molecular weight of the crosslinked hyaluronic acid is 2,500,000 Da or more.

3. The hyaluronic acid hydrogel filler according to claim 1, wherein the hyaluronic acid is crosslinked by a crosslinking agent selected from the group consisting of butanediol diglycidyl ether (1,4-butandiol diglycidyl ether: BDDE), ethylene glycol diglycidyl ether (EGDGE), hexanediol diglycidyl ether (1,6-hexanediol diglycidyl ether), propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, bisepoxypropoxy ethylene (1,2-(bis(2,3-epoxypropoxy)ethylene), pentaerythritol polyglycidyl ether and sorbitol polyglycidyl ether.

4. The hyaluronic acid hydrogel filler according to claim 1, further comprising an anesthetic, preferably lidocaine or its salt.

5. The hyaluronic acid hydrogel filler according to claim 1, wherein the filler is for skin injection, soft-tissue augmentation, anti-wrinkle, soft tissue restore or volume expansion, or contour correction.

6. The hyaluronic acid hydrogel filler according to claim 1, comprising a crosslinking agent at a concentration of 1 to 10 mol%, preferably 1 to 5 mol%, based on 1 mole of N-acetyl-D-glucosamine in the hyaluronic acid or its salt.

7. A preparation method of the hyaluronic acid hydrogel filler according to any one of claims 1 to 6, comprising the following steps:

(a) preparing crosslinked hyaluronic acid hydrogels by adding a hyaluronic acid or its salt, and a crosslinking agent, preferably 1,4-butanediol diglycidyl ether, to an alkaline aqueous solution, preferably an aqueous solution of NaOH, KOH, NaHCO$_3$, LiOH or a combination thereof, and stirring;
(b) cutting the hyaluronic acid hydrogels prepared in the step (a);
(c) preparing a buffer solution;
(d) washing and swelling the hyaluronic acid hydrogels cut in the step (b) using the buffer solution prepared in the step (c);
(e) grinding the washed and swollen hyaluronic acid hydrogels in the step (d); and
(f) filling the hydrogels prepared in the step (e) into a syringe and then sterilizing, in which a hyaluronic acid or its salt is crosslinked,
wherein the filler has properties of both monophasic and biphasic fillers,
the crosslinking ratio of the hyaluronic acid hydrogel filler is 0.1 to 0.2,
the concentration of the crosslinking agent in the step (a) is 1 to 10 mol% based on the unit of the added hyaluronic acid or its salt of 1 mole, , and
the concentration of the hyaluronic acid or its salt is 10 to 25 % by weight as a weight ratio of the hyaluronic acid or its salt based on the total weight of the mixture of the hyaluronic acid or its salt and alkaline aqueous solution.

8. The preparation method according to claim 7, wherein a concentration of the alkaline aqueous solution is 0.1N to 0.5N.

9. The preparation method according to claim 7, wherein the step (a) is performed at a temperature range of 25 to 40 °C for 15 to 22 hours.

10. The preparation method according to claim 7, wherein the buffer solution of the step (c) comprises one or more kinds selected from the group consisting of citric acid, sodium monohydrogen phosphate, sodium dihydrogen phosphate, acetic acid, diethyl barbituric acid, sodium acetate, TAPS (tris(hydroxymethyl)methylamino)propanesulfonic acid, Bicine (2-bis(2-hydroxyethyl)amino)acetic acid, tris(hydroxymethyl)amino methane, Tricine (N-(2-hydroxy-1,1-bis(hydroxymethyl)ethyl)glycine, HEPES (4-(2-hydroxyethyl)-1-piperazine ethanesulphonic acid, TES (2-[[1,3-dihydroxy-2-(hydroxymethyl)propan-2-yl]amino]methanesulfonic acid and PIPES (piperazine-N,N'-bis(2-ethanesulfonic acid), or optionally further comprises an anesthetic and an isotonic agent.

11. A prefilled syringe in which the hyaluronic acid hydrogel filler according to any one of claims 1 to 6 is filled.

12. A biomaterial for soft-tissue augmentation comprising the hyaluronic acid hydrogel filler according to any one of claims 1 to 6.

**Patentansprüche**

1. Hyaluronsäurehydrogelfüllstoff, umfassend eine Hyaluronsäure oder ihr Salz, und zeigend Lifting-Kapazität von 12.000 bis 44.000 Pa*gf, wie gemessen mittels eines Rheometers, und eine Injektionskraft von 15 bis 25 N, wenn sie mittels eines Zugprüfgeräts gemessen wird, wobei die Hyaluronsäure oder ihr Salz vernetzt ist,

wobei der Füllstoff sowohl Eigenschaften von mono-phasigen als auch biphasigen Hyaluronsäurefüllstoffen aufweist,
ein Modifizierungsgrad (MOD) der Hyaluronsäure, wie dargestellt durch folgende Gleichung 1, in dem Bereich von 0,01 bis 0,07 (1 bis 7 %) ist:

[Gleichung 1]

$$\text{Modifizierungsgrad} = \text{Gesamtzahl an Molen an Vernetzungsmittel/Gesamtzahl an Molen von N-Acetyl-D-glucosamin, und}$$

ein Vernetzungsverhältnis (CrR), wie dargestellt durch die folgende Gleichung 2, im Bereich von 0,1 bis 0,2 ist:

[Gleichung 2]

$$\text{Vernetzungsverhältnis} = \text{Anzahl an Molen an vernetztem Vernetzungsmittel/Anzahl an Molen an gesamtem Vernetzungsmittel.}$$

2.  Hyaluronsäurehydrogelfüllstoff nach Anspruch 1, wobei das Molekulargewicht der vernetzten Hyaluronsäure 2.500.000 Da oder höher ist.

3.  Hyaluronsäurehydrogelfüllstoff nach Anspruch 1, wobei die Hyaluronsäure durch ein Vernetzungsmittel vernetzt ist, das ausgewählt ist aus der Gruppe bestehend aus Butandioldiglycidylether (1,4-Butandioldiglycidylether: BDDE), Ethylenglycoldiglycidylether (EGDGE), Hexandioldiglycidylether (1,6-Hexandioldiglycidylether), Propylenglycoldiglycidylether, Polypropylenglycoldiglycidylether, Polytetramethylenglycoldiglycidylether, Neopentylglycoldiglycidylether, Polyglycerolpolyglycidylether, Diglycerolpolyglycidylether, Glycerolpolyglycidylether, Trimethylpropanpolyglycidylether, Bisepoxypropoxyethylen (1,2-(bis(2,3-Epoxypropoxy)ethylen), Pentaerythritolpolyglycidylether und Sorbitolpolyglycidylether.

4.  Hyaluronsäurehydrogelfüllstoff nach Anspruch 1, weiter umfassend ein Anästhetikum, bevorzugt Lidocain oder sein Salz.

5.  Hyaluronsäurehydrogelfüllstoff nach Anspruch 1, wobei der Füllstoff zur Hautinjektion, Weichgewebeaugmentation, Antifalten, Weichgewebewiederherstellung oder Volumenexpansion oder Konturkorrektur ist.

6.  Hyaluronsäurehydrogelfüllstoff nach Anspruch 1, umfassend ein Vernetzungsmittel mit einer Konzentration von 1 bis 10 Mol-%, bevorzugt 1 bis 5 Mol-%, basierend auf 1 Mol N-Acetyl-D-gucosamin in der Hyaluronsäure oder ihrem Salz.

7.  Herstellungsverfahren des Hyaluronsäurehydrogelfüllstoffs nach einem der Ansprüche 1 bis 6, umfassend die folgenden Schritte:

    (a) Herstellen von vernetzten Hyaluronsäurehydrogelen durch Zufügen einer Hyaluronsäure oder ihres Salzes und eines Vernetzungsmittels, bevorzugt 1,4-Butandioldiglycidylether, zu einer alkalischen wässrigen Lösung, bevorzugt einer wässrigen Lösung von NaOH, KOH, NaHCO$_3$, LiOH oder einer Kombination derselben, und Rühren;
    (b) Schneiden der in Schritt (a) hergestellten Hyaluronsäurehydrogele;
    (c) Herstellen einer Pufferlösung;
    (d) Waschen und Quellen der im Schritt (b) geschnittenen Hyaluronsäurehydrogele unter Verwendung der in Schritt (c) hergestellten Pufferlösung;
    (e) Zerkleinern der im Schritt (d) gewaschenen und gequollenen Hyaluronsäurehydrogele; und
    (f) Füllen der im Schritt (e) hergestellten Hydrogele in eine Spritze und dann Sterilisieren, wobei eine Hyaluronsäure oder ihr Salz vernetzt ist,

        wobei der Füllstoff Eigenschaften von sowohl mono-phasigen als auch biphasigen Füllstoffen aufweist, das Vernetzungsverhältnis des Hyaluronsäurehydrogelfüllstoffs 0,1 bis 0,2 ist,

die Konzentration des Vernetzungsmittels im Schritt (a) 1 bis 10 Mol-% ist, basierend auf der Einheit der zugefügten Hyaluronsäure oder ihres Salzes von 1 Mol, und

die Konzentration der Hyaluronsäure oder ihres Salzes 10 bis 25 Gew.-% ist, als ein Gewichtsverhältnis der Hyaluronsäure oder ihres Salzes, basierend auf dem Gesamtgewicht der Mischung der Hyaluronsäure oder ihres Salzes und der alkalischen wässrigen Lösung.

**8.** Herstellungsverfahren nach Anspruch 7, wobei eine Konzentration der alkalischen wässrigen Lösung 0,1 N bis 0,5 N ist.

**9.** Herstellungsverfahren nach Anspruch 7, wobei der Schritt (a) bei einem Temperaturbereich von 25 bis 40°C für 15 bis 22 Stunden durchgeführt wird.

**10.** Herstellungsverfahren nach Anspruch 7, wobei die Pufferlösung des Schritts (c) eine oder mehrere Arten umfasst, ausgewählt aus der Gruppe bestehend aus Citronensäure, Natriummonohydrogenphosphat, Natriumdihydrogenphosphat, Essigsäure, Diethylbarbitursäure, Natriumacetat, TAPS (Tris(hydroxymethyl)methylamino)propansulfonsäure, Bicin (2-bis(2-Hydroxyethyl)amino)essigsäure, Tris(hydroxymethyl)aminomethan, Tricin (N-(2-Hydroxy-1,1-bis(hydroxymethyl)ethyl)glycin, HEPES (4-(2-Hydroxyethyl)-1-piperazinethansulfonäure, TES (2-[[1,3-Dihydroxy-2-(hydroxymethyl)propan-2-yl]amino]methansulfonsäure und PIPES (Piperazin-N,N'-bis(2-ethansulfonsäure), oder optional weiter ein Anästhetikum und ein isotonisches Mittel umfasst.

**11.** Vorgefüllte Spritze, in die der Hyaluronsäurehydrogelfülstoff nach einem der Ansprüche 1 bis 6 eingefüllt ist.

**12.** Biomaterial zur Weichgewebeaugmentation, das den Hyaluronsäurehydrogelfüllstoff nach einem Ansprüche 1 bis 6 umfasst.

## Revendications

**1.** Charge hydrogel d'acide hyaluronique, comprenant un acide hyaluronique ou son sel et présentant une capacité de levage comprise entre 12 000 et 44 000 Pa*gf, mesurée par un rhéomètre et une force d'injection comprise entre 15 et 25 N, mesurée par une machine d'essai de traction, dans laquelle l'acide hyaluronique ou son sel est réticulé, dans laquelle

la charge a à la fois des propriétés de charges d'acide monophasique et biphasique,
un degré de modification (MOD) de l'acide hyaluronique représenté par l'Équation 1 ci-après est compris entre 0,01 et 0,07 (entre 1 et 7 %) :

[Équation 1]

Degré de modification = nombre total de moles de l'agent de réticulation

/ nombre total de moles de N-acétyle-D-glucosamine, et

un taux de réticulation (CrR) représenté par l'Équation 2 ci-après est compris entre 0,1 et 0,2 :

[Équation 2]

Taux de réticulation = nombre de moles de l'agent de réticulation

réticulé / nombre de moles de l'agent de réticulation total.

**2.** Charge hydrogel d'acide hyaluronique selon la revendication 1, dans laquelle le poids moléculaire de l'acide hyaluronique réticulé est égal ou supérieur à 2 500 000 Da.

**3.** Charge hydrogel d'acide hyaluronique selon la revendication 1, dans laquelle l'acide hyaluronique est réticulé par un agent de réticulation sélectionné dans le groupe constitué de l'éther diglycidylique de butanediol (éther diglycidylique de 1,4-butandiol : BDDE), éther diglycidylique d'éthylène glycol (EGDGE), éther diglycidylique d'hexanediol (éther diglycidylique de 1,6-hexanediol), éther diglycidylique de propylène glycol, éther diglycidylique de polypropylène glycol, éther diglycidylique de polytétraméthylène glycol, éther diglycidylique de néopentyle glycol, éther polyglycidylique de polyglycérol, éther polyglycidylique de diglycérol, éther polyglycidylique de glycérol, éther polyglycidylique de tri-méthylpropane, bis(époxypropoxy) éthylène (1,2-(bis(2,3-époxypropoxy)éthylène), éther polyglycidylique de pentaérythritol et éther polyglycidylique de sorbitol.

**4.** Charge hydrogel d'acide hyaluronique selon la revendication 1, comprenant en outre un anesthésiant, de préférence de la lidocaïne ou son sel.

**5.** Charge hydrogel d'acide hyaluronique selon la revendication 1, dans laquelle la charge est destinée à une injection dans la peau, une augmentation des tissus mous, un anti-rides, une restauration ou augmentation du volume des tissus mous ou une correction des contours.

**6.** Charge hydrogel d'acide hyaluronique selon la revendication 1, comprenant un agent de réticulation à une concentration comprise entre 1 et 10 % en moles, de préférence entre 1 et 5 % en moles, sur la base de 1 mole de N-acétyle-D-glucosamine dans l'acide hyaluronique ou son sel.

**7.** Procédé de préparation de la charge hydrogel d'acide hyaluronique selon l'une quelconque des revendications 1 à 6, comprenant les étapes suivantes consistant à :

(a) préparer des hydrogels d'acide hyaluronique réticulés en ajoutant un acide hyaluronique ou son sel, et un agent de réticulation, de préférence un éther diglycidylique de 1,4-butanediol, à une solution aqueuse alcaline, de préférence une solution aqueuse de NaOH, KOH, NaHCO$_3$, LiOH ou une combinaison de ces derniers, et en remuant ;
(b) couper les hydrogels d'acide hyaluronique préparés à l'étape (a) ;
(c) préparer une solution tampon ;
(d) laver et gonfler les hydrogels d'acide hyaluronique coupés à l'étape (b) en utilisant la solution tampon préparée à l'étape (c) ;
(e) broyer les hydrogels d'acide hyaluronique lavés et gonflés à l'étape (d) ; et
(f) remplir les hydrogels préparés à l'étape (e) dans une seringue, puis stériliser, dans lequel un acide hyaluronique ou son sel est réticulé,
dans lequel la charge a des propriétés à la fois de charges monophasique et biphasique,
le taux de réticulation de la charge hydrogel d'acide hyaluronique est compris entre 0,1 et 0,2,
la concentration de l'agent de réticulation à l'étape (a) est comprise entre 1 et 10 % en moles sur la base de l'unité de l'acide hyaluronique ou de son sel ajouté de 1 mole, et
la concentration de l'acide hyaluronique ou de son sel est comprise entre 10 et 25 % en poids en rapport de poids de l'acide hyaluronique ou de son sel sur la base du poids total du mélange de l'acide hyaluronique ou de son sel et de la solution aqueuse alcaline.

**8.** Procédé de préparation selon la revendication 7, dans lequel une concentration de la solution aqueuse alcaline est comprise entre 0,1 N et 0,5 N.

**9.** Procédé de préparation selon la revendication 7, dans lequel l'étape (a) est effectuée à une température comprise entre 25 et 40°C pendant 15 à 22 heures.

**10.** Procédé de préparation selon la revendication 7, dans lequel la solution tampon de l'étape (c) comprend une ou plusieurs sortes sélectionnées dans le groupe constitué de l'acide citrique, phosphate monohydrogène de sodium, phosphate dihydrogène de sodium, acide acétique, acide barbiturique diéthyle, acétate de sodium, acide (tris(hydroxyméthyl)méthylamino)propanesulfonique TAPS, acide (2-bis(2-hydroxyéthyl)amino)acétique Bicine, tris(hydroxyméthyl)aminométhane, (N-(2-hydroxy-1,1-bis(hydroxyméthyl)éthyl)glycine Tricine, acide (4-(2-hydroxyéthyl)-1-pipérazine éthanesulfonique HEPES, acide (2-[[1,3-dihydroxy-2-(hydroxyméthyl)propane-2-yl]amino]méthanesulfonique TES et acide pipérazine-N,N'-bis(2-éthanesulfonique) PIPES ou, en option, comprend en outre un anesthésiant et un agent isotonique.

**11.** Seringue pré-remplie dans laquelle la charge hydrogel d'acide hyaluronique selon l'une quelconque des revendications 1 à 6 est remplie.

12. Biomatériau permettant l'augmentation des tissus mous comprenant la charge hydrogel d'acide hyaluronique selon l'une quelconque des revendications 1 à 6.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020180080210
- US 4582865 A
- US 5827937 A
- WO 2016074794 A